(19) Europäisches Patentamt
European Patent Office
Office européen des brevets

(11) Numéro de publication : **0 668 273 A1**

# (12) DEMANDE DE BREVET EUROPEEN

(21) Numéro de dépôt : **95400304.2**

(51) Int. Cl.[6] : **C07D 295/08**, A61K 31/495

(22) Date de dépôt : **14.02.95**

(30) Priorité : **16.02.94 FR 9401736**

(43) Date de publication de la demande : **23.08.95 Bulletin 95/34**

(84) Etats contractants désignés : **AT BE CH DE DK ES FR GB GR IE IT LI LU MC NL PT SE**

(71) Demandeur : **SYNTHELABO
22, Avenue Galilée
F-92350 Le Plessis Robinson (FR)**

(72) Inventeur : **Manoury, Philippe
L'Orée de Verrières,
38 avenue des Vaupépins
F-91370 Verrières le Buisson (FR)**
Inventeur : **Obitz, Daniel
64 rue Velpeau
F-92160 Antony (FR)**
Inventeur : **Peynot, Michel
2 rue des Marguerites
F-94240 L'Hay Les Roses (FR)**
Inventeur : **Sevrin, Mireille
73 rue Raymond Losserand
F-75014 Paris (FR)**
Inventeur : **George, Pascal
19 rue des Quatre Vents
F-78730 St Arnoult en Yvelines (FR)**

(74) Mandataire : **Ludwig, Jacques et al
SYNTHELABO,
Service Brevets,
B.P. 72
F-92352 Le Plessis Robinson Cédex (FR)**

(54) **Dérivés de 1-[2-(1H-indén-3-yl)éthyl]-4-(naphtalén-1-yl)-pipérazine, leur préparation et leur application en thérapeutique.**

(57) Composés de formule générale (I)

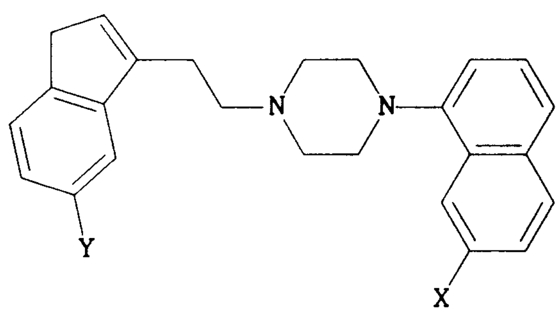

(I)

dans laquelle X représente un atome d'hydrogène, un groupe alcoxy en $C_1$-$C_3$ ou un groupe cyclopropylméthoxy, et Y représente un atome d'hydrogène ou un groupe méthoxy.
Application en thérapeutique.

EP 0 668 273 A1

La présente invention a pour objet des dérivés de 1-[2-(1*H*-indén-3-yl)éthyl]-4-(naphtalén-1-yl)pipérazine, leur préparation et leur application en thérapeutique.

Les composés de l'invention répondent à la formule générale (I)

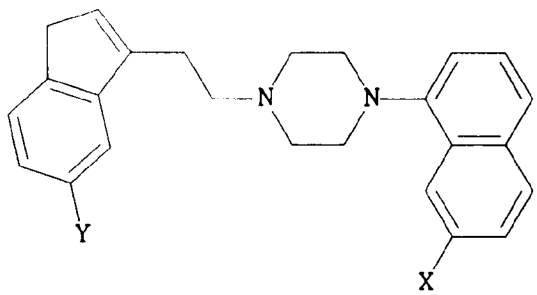

(I)

dans laquelle
X représente un atome d'hydrogène, un groupe alcoxy en $C_1$-$C_3$ ou un groupe cyclopropylméthoxy, et
Y représente un atome d'hydrogène ou un groupe méthoxy.

X représente de préférence un groupe alcoxy en $C_1$-$C_3$, en particulier un groupe méthoxy, et Y représente de préférence un groupe méthoxy.

Les composés selon l'invention peuvent exister à l'état de bases ou de sels d'addition.

Des composés de structure chimique analogue à celle des composés de formule générale (I), et qui sont utilisables comme agents antidépresseurs et anxiolytiques, sont décrits dans la demande de brevet EP-0490772.

Conformément à l'invention, on peut préparer les composés de formule générale (I) par des procédés illustrés par les schémas 1 et 2 qui suivent.

Selon le schéma 1, on traite un dérivé d'acide 1*H*-indène-3-acétique de formule générale (II), dans laquelle Y est tel que défini ci-dessus, avec un agent réducteur, simple ou complexe, tel qu'un hydrure alcalin ou métallique, par exemple l'hydrure de lithium et d'aluminium, l'hydrure de bore, le complexe hydrure de bore-tétrahydrofurane ou hydrure de bore-sulfure de diméthyle ou l'hydrure

Schéma 1

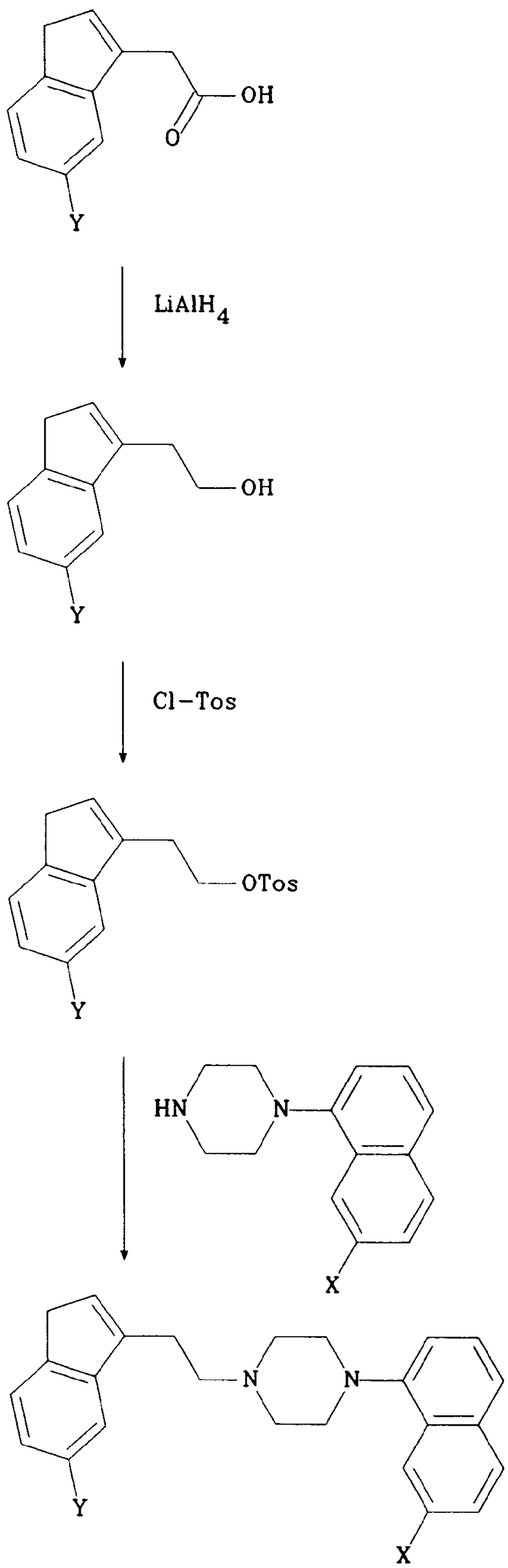


(II)

(III)

(IV)

(V)

(I)

d'aluminium, dans un solvant inerte, aromatique ou éthéré, par exemple le toluène, le xylène, l'éther diéthylique, le tétrahydrofurane, le dioxane, à une température de 30 à 140°C, selon le solvant, pour former l'alcool de formule générale (III).On traite ensuite cet alcool par le chlorure d'acide 4-méthylbenzènesulfonique, en présence d'une base organique telle que la triéthylamine ou la pyridine, et éventuellement en présence d'un solvant inerte, à une température de 0 à 40°C, pour obtenir le dérivé de formule générale (IV).

Finalement on fait réagir ce dernier avec un dérivé de pipérazine de formule générale (V), dans laquelle X est tel que défini ci-dessus, à une température de 100 à 150°C, de préférence à 130°C, éventuellement dans un solvant à point d'ébullition élevé, tel que le toluène, le xylène, le *N*,*N*-diméthylformamide ou la 1-méthylpyrrolidin-2-one.

Selon le procédé illustré par le schéma 2, on fait réagir le dérivé d'acide 1*H*-indène-3-acétique de formule générale (II), dans laquelle Y est tel que défini ci-dessus, d'abord avec le *N*,*N'*-carbonyldiimidazole, dans un solvant inerte, tel que le tétrahydrofurane, à une température de 20 à 50°C, pour obtenir *in situ* l'imidazolide correspondant, puis on traite ce dernier avec un dérivé de pipérazine de formule générale (V), dans laquelle X est tel que défini ci-dessus, dans un solvant inerte, tel qu'un solvant éthéré, par exemple le tétrahydrofurane ou le dioxane, à une température de 20 à 50°C, pour obtenir l'amide de formule générale (VI). Finalement on réduit ce dernier au moyen d'un agent réducteur simple ou complexe, tel qu'un hydrure alcalin ou métallique, par exemple l'hydrure de lithium et d'aluminium, l'hydrure de bore, le complexe hydrure de bore-tétrahydrofurane ou hydrure de bore-sulfure de diméthyle, l'hydrure d'aluminium, dans un solvant inerte, aromatique ou éthéré, par exemple le toluène, le xylène, l'éther diéthylique, le tétrahydrofurane, le dioxane, à une température de 30 à 140°C, selon le solvant.

Schéma 2

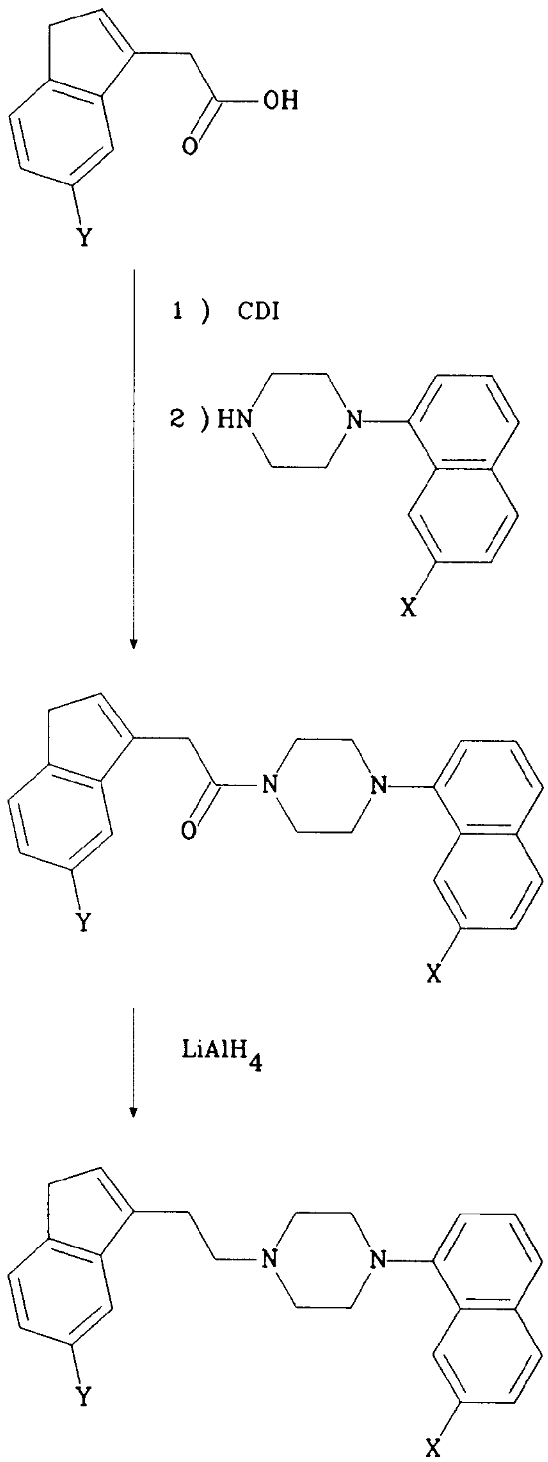


(II)

(V)

(VI)

(I)

Les composés de départ de formule générale (II) sont décrits dans *C.A.* **76**(23) 140279s, *C.A.* **104**(1) 5652q et *J. Chem. Soc. Perkin Trans.* (1972) **1**(7) 941 : on traite la 2,3-dihydro-1*H*-indén-1-one (Y=H, disponible dans le commerce) ou la 6-méthoxy-2,3-dihydro-1*H*-indén-1-one (Y=$OCH_3$, décrite dans *J. Org. Chem.* (1970) **35**(3) 647 et *J. Org. Chem* (1977) **42**(12) 2155) par le bromoacétate d'éthyle en présence de zinc en poudre dans les conditions de la réaction de Reformatsky, pour obtenir un mélange de (6-Y-2,3-dihydro-1*H*-indén-1-ylidène)acétate d'éthyle et de 5-Y-1*H*-indène-3-acétate d'éthyle. L'hydrolyse de ce mélange en milieu alcoolique basique fournit ensuite l'acide de formule générale (II).

Les dérivés de pipérazine de formule générale (V) sont connus et peuvent être obtenus par des méthodes décrites dans la littérature, par exemple dans les demandes de brevets EP-0343050, EP-0354093 et EP-0434561, dans *J. Med. Chem.* (1986) **29**(11) 2379, *J. Med. Chem.* (1988) **31**(10) 1968 et dans *J. Med. Chem.* (1991) **34**(8) 2623.

Les exemples qui suivent illustrent en détail la préparation des composés selon l'invention.
Les microanalyses élémentaires et les spectres IR et RMN confirment les structures des produits obtenus.
Les numéros indiqués entre parenthèses dans les titres des exemples correspondent à ceux de la première colonne du tableau donné plus loin.

Exemple 1 (Composé N°4)

(*E*)-2-Butènedioate (1:2) de 4-[2-(5-méthoxy-1*H*-indén-3-yl)éthyl]-1-(7-méthoxynaphtalén-1-yl)pipérazine.

1.1. 5-Méthoxy-1*H*-indène-3-éthanol.

On prépare une suspension de 0,76 g (0,02 mole) d'hydrure de lithium et d'aluminium dans 50 ml d'éther diéthylique, on ajoute une solution de 2,04 g (0,01 mole) d'acide 5-méthoxy-1*H*-indène-3-acétique, on agite le mélange et on le chauffe au reflux pendant 32h.

On laisse refroidir le mélange, on l'hydrolyse avec 1,6 ml de solution aqueuse à 10% de tartrate double de potassium et de sodium, on le rechauffe à l'ébullition pendant 1h, on le filtre, en rincant le résidu avec du tétrahydrofurane, et on évapore le filtrat sous pression réduite.

On obtient 1,8 g de résidu huileux qu'on purifie par distillation.

On obtient 1,55 g de liquide jaune qu'on utilise tel quel dans l'étape suivante.

1.2. 4-Méthylbenzènesulfonate de 2-(5-méthoxy-1*H*-indén-3-yl)éthyle.

On dissout 1,27 g (0,0067 mole) de 5-méthoxy-1*H*-indène-3-éthanol dans 11 ml de pyridine sèche, on agite le mélange, on le refroidit par un bain de glace, on ajoute peu à peu 1,4 g (0,0073 mole) de chlorure d'acide 4-méthylbenzènesulfonique, et on maintient l'agitation à froid pendant une nuit, puis à température ambiante pendant 4h.

On verse la solution sur un mélange de 16 ml d'acide chlorhydrique 10N et 48 g de glace, on traite le mélange obtenu à l'éther diéthylique, on sépare la phase organique, on la lave à l'eau, on la sèche sur sulfate de magnésium, on la filtre et on évapore le filtrat sous pression réduite.

On obtient 1,94 g de produit huileux incolore qu'on utilise tel quel dans l'étape suivante.

1.3. (*E*)-2-Butènedioate (1:2) de 4-[2-(5-méthoxy-1*H*-indén-3-yl)éthyl]-1-(7-méthoxynaphtalén-1-yl)pipérazine.

On mélange 2,07 (0,006 mole) de 4-méthylbenzènesulfonate de 2-(5-méthoxy-1*H*-indén-3-yl)éthyle et 2,90 g (0,012 mole) de 1-(7-méthoxynaphtalén-1-yl)pipérazine, on agite le mélange, on le place sous atmosphère d'argon et on le chauffe au bain d'huile à 130°C pendant 2h.

On reprend le mélange avec du dichlorométhane, on lave la solution à l'eau, à la soude diluée, puis de nouveau à l'eau, on la sèche sur sulfate de magnésium, on la filtre, on évapore le filtrat sous pression réduite.

On obtient 4,08 g d'huile qu'on purifie par chromatographie sur colonne de gel de silice en éluant avec un mélange de dichlorométhane/acétone 92/8.

On obtient 2,09 g de base dont on dissout 2,03 g (0,0049 mole) dans un mélange de propan-2-ol et d'éther diéthylique, on tiédit la solution, on ajoute une solution de 0,569 g d'acide fumarique dans du propan-2-ol chaud, on laisse refroidir en agitant et on laisse reposer pendant une nuit.

On obtient finalement 2,16 g de fumarate neutre.

Point de fusion : 158-159°C.

Exemple 2 (Composé N°2)

4-[2-(5-Méthoxy-1*H*-indén-3-yl)éthyl]-1-(naphtalén-1-yl)pipérazine.

2.1. 4-[(5-Méthoxy-1*H*-indén-3-yl)acétyl]-1-(naphtalén-1-yl)pipérazine.

A une solution de 2,45 g (0,012 mole) d'acide 5-méthoxy-1*H*-indène-3-acétique dans 12 ml de tétrahydrofurane, placée sous atmosphère d'argon, on ajoute, par petites portions, 2,0 g (0,012 mole) de *N,N'*-carbonyldiimidazole, et on agite le mélange pendant 1h.

On ajoute une solution de 2,55 g (0,012 mole) de 1-(naphtalén-1-yl)pipérazine dans 10 ml de tétrahydrofurane, et on laisse le mélange au repos pendant une nuit.

On évapore le solvant sous pression réduite, on reprend l'huile résiduelle avec de l'eau et de l'éther diéthylique, on collecte le solide obtenu par filtration et on le sèche.

On obtient 4,13 g de produit qu'on utilise tel quel dans l'étape suivante.

2.2. 4-[2-(5-Méthoxy-1*H*-indén-3-yl)éthyl]-1-(naphtalén-1-yl)pipérazine.

Dans un ballon de 500 ml, sous atmosphère d'argon, on place 0,76 g (0,02 mole) d'hydrure de lithium et d'aluminium, on le recouvre d'éther diéthylique, on surmonte le ballon d'un extracteur de Soxhlet contenant 2,0 g (0,005 mole) de 4-[(5-méthoxy-1*H*-indén-3-yl)acétyl]-1-(naphtalén-1-yl)pipérazine, et on laisse réagir en faisant refluer l'éther pendant 30h.

On traite le mélange réactionnel avec 1,6 ml de solution aqueuse à 10% de tartrate double de potassium et de sodium, on filtre le mélange, en lavant le solide à l'éther diéthylique puis au tétrahydrofurane, et on évapore le filtrat sous pression réduite.

On obtient une huile qui cristallise. Après traitement à l'éther diéthylique et recristallisation dans un mélange d'hexane et d'éther diisopropylique on obtient finalement 0,45 g de composé.

Point de fusion : 102-103°C.

Exemple 3 (Composé N°3)

(*E*)-2-Butènedioate (1:1) de 4-[2-(1*H*-indén-3-yl)éthyl]-1-(7-méthoxynaphtalén-1-yl)pipérazine.

3.1. 1*H*-indène-3-éthanol.

On prépare une suspension de 3,4 g (0,09 mole) d'hydrure de lithium et d'aluminium dans 200 ml d'éther diéthylique sec, on ajoute, goutte à goutte, une solution de 7,7 g (0,044 mole) d'acide 1*H*-indène-3-acétique dans 150 ml d'éther diéthylique, on agite le mélange et on le chauffe au reflux pendant 20h.

On laisse refroidir le mélange, on l'hydrolyse avec environ 8 ml de solution aqueuse à 10% de tartrate double de potassium et de sodium, on le rechauffe à l'ébullition pendant 1h, on le filtre, en rincant le résidu avec de l'éther diéthylique, on évapore le filtrat sous pression réduite et on purifie le résidu par distillation.

On obtient 5,2 g de produit qu'on utilise tel quel dans l'étape suivante.

3.2. 4-Méthylbenzènesulfonate de 2-(1*H*-indén-3-yl)éthyle.

On dissout 5 g (0,031 mole) de 1*H*-indène-3-éthanol dans 50 ml de pyridine sèche, on agite le mélange, on le refroidit par un bain de glace, on ajoute peu à peu 5,9 g (0,031 mole) de chlorure d'acide 4-méthylbenzènesulfonique, et on maintient l'agitation à froid pendant 1h puis à température ambiante pendant 4h.

On verse la solution sur un mélange de 100 ml d'acide chlorhydrique 10N et 200 g de glace, on extrait le mélange deux fois à l'éther diéthylique, on sépare la phase organique, on la lave à l'eau, on la sèche sur sulfate de magnésium, on la filtre et on évapore le filtrat sous pression réduite.

On obtient 7 g de produit huileux qu'on utilise tel quel dans l'étape suivante.

3.3. (*E*)-2-Butènedioate (1:1) de 4-[2-(1*H*-indén-3-yl)éthyl]-1-(7-méthoxynaphtalén-1-yl)pipérazine.

On mélange 1,15 g (0,00366 mole) de 4-méthylbenzènesulfonate de 2-(1*H*-indén-3-yl)éthyle et 1,95 g (0,008 mole) de 1-(7-méthoxynaphtalén-1-yl)pipérazine, on agite le mélange, on le place sous atmosphère d'argon et on le chauffe au bain d'huile à 130°C pendant 3h.

On laisse refroidir le mélange, on le reprend avec 10 ml de soude à 10%, et on l'extrait avec du dichlorométhane. On lave la solution à l'eau, on la sèche sur sulfate de magnésium, on la filtre, on évapore le filtrat sous pression réduite.

On purifie le résidu par chromatographie sur colonne de gel de silice en éluant avec un mélange de dichlorométhane/acétone 98/2.

On obtient 1,3 g (0,00338 mole) de base qu'on dissout dans 50 ml de propan-2-ol, on ajoute 0,4 g d'acide fumarique, on laisse refroidir tout en agitant et on laisse reposer le mélange pendant une nuit.

Après filtration, lavage à l'éther diéthylique, séchage et recristallisation dans l'éthanol, on obtient finalement 1,15 g de fumarate.

Point de fusion : 184-185°C.

Exemple 4 (Composé N°10)

(*E*)-2-Butènedioate (1:1) de 4-[2-(5-méthoxy-1*H*-indén-3-yl)éthyl]-1-[7-(cyclopropylméthoxy)naphtalén-1-yl]pipérazine.

4.1. *N*-(7-hydroxynaphtalén-1-yl)acétamide.

On verse 100 g (0,55 mole) de 8-aminonaphtalén-2-ol dans 125 ml (135,25 g, soit 1,325 mole) d'anhydride acétique refroidi par un bain de glace, et on agite le mélange à froid pendant 1h.

On verse le mélange dans 375 ml d'eau glacée, on l'agite pendant plusieurs heures, on collecte le solide violet par filtration, on le lave trois fois avec 30 ml d'éther diéthylique et on le sèche sous pression réduite.

On obtient 108,8 g de produit qu'on utilise tel quel dans l'étape suivante.

Point de fusion : 193-195°C.

4.2. *N*-[7-(Cyclopropylméthoxy)naphtalén-1-yl]acétamide.

A une suspension de 3,4 g (0,085 mole) d'hydrure de sodium à 60% dans l'huile, préalablement lavé dans du pentane sec, dans 100 ml de diméthylsulfoxyde on ajoute, sous atmosphère d'azote, 17,1 g (0,085 mole) de *N*-(7-hydroxynaphtalén-1-yl)acétamide en solution dans 50 ml de diméthylsulfoxyde, en refroidissant le mélange par un bain d'eau glacée, et on maintient l'agitation à température ambiante pendant 2h.

On ajoute 9,05 g (0,1 mole) de (chlorométhyl)cyclopropane, on agite à température ambiante pendant 4h et on laisse reposer le mélange pendant une nuit.

On verse le mélange dans 1 l d'eau, on l'agite pendant 1h et on le laisse reposer au froid pendant une nuit.

On sépare le solide par filtration, on le lave à l'eau et on le sèche. On obtient 13 g de produit qu'on utilise tel quel dans l'étape suivante.

Point de fusion : 154-155°C.

4.3. 7-(Cyclopropylméthoxy)naphtalène-1-amine.

Sous atmosphère d'azote on chauffe au reflux un mélange de 13 g (0,05 mole) de *N*-[7-(cyclopropylméthoxy)naphtalén-1-yl]acétamide, 35 ml de soude 10N et 150 ml de 2-méthoxy éthanol, pendant 4h.

On évapore le solvant sous pression réduite, on reprend le résidu avec 200 ml de dichlorométhane et 200 ml d'eau, on agite le mélange en présence de noir de charbon, on le filtre sur kieselguhr, on sépare la phase organique, on la sèche sur sulfate de magnésium, on la filtre, on évapore le solvant sous pression réduite et on purifie le résidu par chromatographie sur colonne de gel de silice en éluant avec du dichlorométhane.

On obtient 7,8 g d'une huile qui cristallise en refroidissant.

Point de fusion : 49-50°C.

4.4. 1-[7-(Cyclopropylméthoxy)naphtalén-1-yl]pipérazine.

Sous atmosphère d'azote on chauffe au reflux un mélange de 7,7 g (0,036 mole) de 7-(cyclopropylméthoxy)naphtalène-1-amine, 6 g (0,036 mole) de chlorhydrate de bis(2-chloroéthyl)amine, 50 ml de butanol et environ 50 mg d'iodure de potassium, pendant 10h.

On ajoute 2,5 g (0,018 mole) de carbonate de potassium et on poursuit le chauffage au reflux pendant 10h, puis, à deux reprises, on ajoute encore 1,25 g (0,09 mole) de carbonate de potassium et on chauffe au reflux pendant 10h.

On évapore le butanol, on reprend le résidu avec 100 ml de dichlorométhane et 50 ml de soude à 10%, on agite le mélange en présence de noir de charbon, on le filtre, on sépare la phase organique, on la sèche sur sulfate de magnésium, on évapore le solvant sous pression réduite et on purifie le résidu par chromatographie sur colonne de gel de silice en éluant avec un mélange de dichlorométhane/méthanol 90/10.

Après évaporation du solvant on obtient 4,2 g de produit huileux qu'on utilise tel quel dans l'étape suivante.

4.5. (*E*)-2-Butènedioate (1:1) de 4-[2-(5-méthoxy-1*H*-indén-3-yl)éthyl]-1-[7-(cyclopropylméthoxy)naphtalén-1-yl]pipérazine.

Sous atmosphère d'azote on chauffe progressivement un mélange de 1,14 g (0,0033 mole) de 4-méthylbenzènesulfonate de 2-(5-méthoxy-1*H*-indén-3-yl)éthyle et 2 g (0,007 mole) de 1-[7-(cyclopropylméthoxy)naphtalén-1-yl]pipérazine, et on maintient le chauffage à 130°C pendant 3h.

On reprend le mélange avec 20 ml de soude à 10% et on l'extrait au dichlorométhane. On sèche la phase organique sur sulfate de magnésium, on évapore le solvant sous pression réduite et on purifie le résidu par chromatographie sur colonne de gel de silice en éluant avec un mélange dichlorométhane/acétone 98/2.

On obtient 1,3 g de (0,00286 mole) base qu'on dissout dans 20 ml de propan-2-ol, on chauffe la solution au reflux et on ajoute 0,33 g (0,00286 mole) d'acide fumarique. On laisse refroidir, on sépare le solide par filtration, on le recristallise dans l'éthanol, on le lave à l'éther diéthylique et on le sèche.

On isole finalement 0,65 g de fumarate.

Point de fusion : 154-155°C.

Le tableau qui suit illustre les structures chimiques et les propriétés physiques de quelques composés selon l'invention.

Dans la colonne "X", "$OCH_2cC_3H_5$" désigne un groupe cyclopropylméthoxy.

Dans la colonne "Sel", "-" désigne un composé à l'état de base, "fum." désigne un fumarate, ou (*E*)-2-butènedioate ; le rapport indiqué entre parenthèses est le rapport molaire acide:base.

Tableau

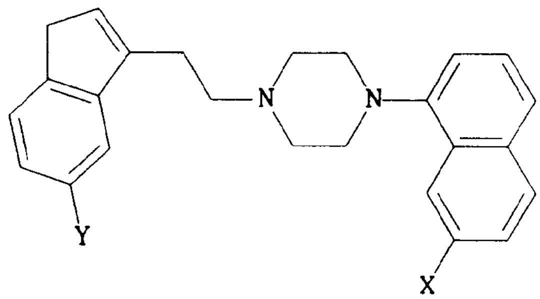

(I)

| N° | X | Y | Sel | F (°C) |
|---|---|---|---|---|
| 1 | H | H | fum. (1:1) | 224-225 |
| 2 | H | $OCH_3$ | - | 102-103 |
| 3 | $OCH_3$ | H | fum. (1:1) | 184-185 |
| 4 | $OCH_3$ | $OCH_3$ | fum. (1:2) | 158-159 |
| 5 | $OCH_2CH_3$ | H | fum. (1:1) | 183-184 |
| 6 | $OCH_2CH_3$ | $OCH_3$ | fum. (1:1) | 142-143 |
| 7 | $OCH_2CH_2CH_3$ | $OCH_3$ | fum. (1:1) | 163-164 |
| 8 | $OCH(CH_3)_2$ | $OCH_3$ | fum. (1:1) | 200-201 |
| 9 | $OCH_2cC_3H_5$ | H | fum. (1:1) | 205-206 |
| 10 | $OCH_2cC_3H_5$ | $OCH_3$ | fum. (1:1) | 154-155 |

Les composés de l'invention ont fait l'objet d'essais qui ont mis en évidence leur intérêt comme substances thérapeutiques.

Ainsi ils ont été testés *in vitro* quant à leur affinité pour les récepteurs sérotoninergiques du type $5\text{-}HT_{1A}$, présents dans l'hippocampe du rat, selon un protocole décrit par Sanger et Schoemaker, *Psychopharmacology* (1992) **108** 85-92.

Les composés déplacent la liaison d'un ligand spécifique marqué, la [$^3$H]-8-hydroxy-2-(di-n-propylamino)tétraline (désignée ci-après par "[$^3$H]-8-OH-DPAT" et décrite par Gozlan et coll., *Nature* (1983) **305** 140-142) sur les récepteurs $5\text{-}HT_{1A}$.

Les animaux utilisés sont des rats mâles Sprague-Dawley de 160 à 200 g. Après décapitation on en prélève le cerveau et on excise l'hippocampe. On broie le tissu dans un appareil Ultra-Turrax Polytron™ pendant 30 s à la moitié de la vitesse maximale dans 10 volumes de tampon Tris 50 mM d'un pH ajusté à 7,4 avec de l'acide chlorhydrique (soit 100 mg de tissu frais par ml). On lave les tissus homogénéisés trois fois à 4°C, en les centrifugeant à chaque fois pendant 10 min à 48000×g et en remettant le culot en suspension dans du tampon frais refroidi. Finalement on met le dernier culot en suspension dans le tampon pour arriver à une concentration de 100 mg de tissu de départ par ml de tampon à 50 mM.

On laisse ensuite incuber à 37°C pendant 10 min.

La liaison avec la [$^3$H]-8-OH-DPAT (1 nM) est déterminée par incubation de 100 µl de suspension de membranes dans un volume final de 1 ml de tampon contenant 10 µM de pargyline et 3 µM de paroxétine.

Après une incubation de 15 min à 37°C on récupère les membranes par filtration sur filtres Whatman GF/B™ qu'on lave trois fois avec des quantités aliquotes de 5 ml de tampon glacé. On extrait les filtres dans le liquide de scintillation et on en mesure la radioactivité par scintigraphie liquide. La liaison spécifique de la [$^3$H]-8-OH-DPAT est définie comme la quantité radioactive retenue sur les filtres et pouvant être inhibée par co-incubation dans la hydroxy-5 tryptamine à 10 μM. A une concentration de 1 nM de [$^3$H]-8-OH-DPAT la liaison spécifique représente 90% de la radioactivité totale recupérée sur le filtre.
Pour chaque concentration de composés étudié on détermine le pourcentage d'inhibition de la liaison avec la [$^3$H]-8-OH-DPAT, puis la concentration $CI_{50}$, concentration qui inhibe 50% de la liaison.
Les $CI_{50}$ se situent entre 10 et 300 nM.

Les composés de l'invention ont aussi fait l'objet d'une étude *in vitro* quant à leur affinité pour les récepteurs sérotoninergiques $5HT_{1D}$ présents dans le noyau caudé de bovin, mise en évidence par le déplacement d'un ligand spécifique marqué, la [$^3$H]-5-hydroxytryptamine, essentiellement comme décrit par Heuring et Peroutka dans *J. Neurosci.*, (1987), **7**, 804-903.
Le noyau caudé de bovin (Collectorgane, Paris) est conservé à -80°C jusqu'à l'utilisation. Le tissu est broyé dans un appareil Ultra-Turrax Polytron™ pendant 30s à la moitié de la vitesse maximale dans 10 volumes de tampon Tris 50mM dont le pH est ajusté à 7,4 avec de l'acide chlorhydrique (soit 100 mg de tissu frais par ml). Les tissus homogénéisés sont lavés deux fois à 4°C et centrifugés pendant 10 min à 40000×g, le culot étant remis à chaque fois en suspension dans du tampon glacé. Finalement le dernier culot est mis en suspension dans le tampon pour arriver à une concentration de 100 mg de tissu de départ par ml de tampon à 50mM, et laissé à incuber à 37°C pendant 15 min. La suspension membranaire est ensuite centrifugée pendant 10 min à 40000×g et le culot est remis en suspension dans 8 volumes de milieu d'incubation contenant du Tris (50mM), de l'acide ascorbique (0,1%), du chlorure de calcium (4mM), de la pargylline (10μM), de la mésulergine (100nM) et de la 8-hydroxy-dipropylamino-tétraline (100nM), et dont le pH est ajusté à 7,4 avec de l'acide chlorhydrique.
La liaison avec la [$^3$H]-5-hydroxytryptamine (2nM) est déterminée par incubation de 100 μl de suspension de membranes dans un volume final de 1 ml de milieu d'incubation.
Après une incubation de 30 min à 37°C suivie d'une incubation de 5 min entre 0 et 4°C, les membranes sont récupérées par filtration sur filtres Whatman GF/B™ qu'on lave deux fois avec des quantités aliquotes de 1ml de tampon Tris 50mM glacé, et dont le pH est ajusté à 7,4 avec de l'acide chlorhydrique.
Les filtres sont extraits dans le liquide de scintillation et la radioactivité est mesurée par scintigraphie liquide. La liaison spécifique de la [$^3$H]-5-hydroxytryptamine est définie comme la quantité de radioactivité retenue sur les filtres et pouvant être inhibée par co-incubation avec la 5-hydroxytryptamine à 0,1μM. A une concentration de 2nM de [$^3$H]-5-hydroxytryptamine la liaison spécifique représente 70% de la radioactivité totale récupérée sur le filtre.
Pour chaque concentration de composé étudié on détermine le pourcentage d'inhibition de la liaison avec la [$^3$H]-5-hydroxytryptamine, puis la concentration $CI_{50}$, concentration qui inhibe 50% de la liaison.
Les composés de l'invention les plus actifs, dans cet essai, ont une $CI_{50}$ inférieure à 30 nM.

Les composés de l'invention ont encore fait l'objet d'un essai *in vitro* de déplacement de la liaison de la spipérone sur les récepteurs sérotoninergiques ($5\text{-}HT_2$) du cortex cérébral du rat.
Pour cet essai on prélève les cerveaux de rats, on en dissèque le cortex et on l'homogénéise à 0°C dans 10 volumes d'un mélange contenant, par litre, 50 millimoles de tampon Tris/HCl à pH = 7,4, 120 millimoles de chlorure de sodium et 5 millimoles de chlorure de potassium. On centrifuge le mélange homogène à 40000×g pendant 10 min puis, à deux reprises, on récupère le culot, on le lave en le mettant en suspension dans le même mélange tampon, on l'homogénéise de nouveau et on le centrifuge. Pour terminer on dilue le culot final dans le même mélange tampon à raison de 100 mg de tissu humide pour 1 ml de tampon.
On soumet alors le tissu à une incubation préalable de 10 min à 37°C en présence de 10 micromoles/l de pargyline, puis à une incubation de 20 min à 37°C en présence de $^3$H-spipérone (activité spécifique : 15 à 30 Ci par millimole) à la concentration de 0,3 nanomole/l et du composé à étudier.

On récupère ensuite les membranes par filtration sur filtres Whatman GF/B™, qu'on lave deux fois avec 5 ml de tampon froid. La radioactivité retenue par le filtre est mesurée par scintigraphie liquide.
Pour évaluer l'activité des composés on établit la courbe du pourcentage d'inhibition de la liaison spécifique de $^3$H-spipérone en fonction de la concentration en drogue déplaçante.
On détermine graphiquement la concentration $CI_{50}$, concentration qui inhibe 50 % de la liaison spécifique.
La liaison spécifique est définie comme étant la liaison déplacée par 100 micromoles/l de 5-HT.
Les concentrations $CI_{50}$ des composés de l'invention se situent entre 50 et 1500 nM.

Enfin les composés de l'invention ont fait l'objet d'une étude *in vitro* quant à leur affinité pour les récepteurs sérotoninergiques $5HT_{1C}$ présents dans le plexus choroïdien de porc, mise en évidence par le déplacement de la liaison d'un ligand spécifique marqué, la [$^3$H]mésulergine, essentiellement comme décrit par Pazos et coll. dans *Eur. J. Pharmacol.*, (1984), **106**, 539-546, et par Yagalof et Hartig dans *Mol. Pharmacol.*, (1986),

**26**, 120-125.
Le plexus choroïdien (Collectorgane, Paris) est conservé à -80°C jusqu'à l'utilisation. Le tissu est homogénéisé dans un homogénisateur Potter™ par 10 à 15 mouvements (800 tpm) dans 10 volumes de saccharose (0,32M) à une température de 0 à 4°C. La suspension membranaire est centrifugée pendant 10 min à 1000×g (4°C) et le surnageant est centrifugé pendant 20 min à 30000×g (4°C). Le culot est mis en suspension dans 10 volumes de tampon Tris 50mM d'un pH ajusté à 7,4 avec de l'acide chlorhydrique, et est ensuite incubé à 37°C pendant 15 min. Finalement la suspension est centrifugée pendant 20 min à 30000×g (4°C), et le culot est repris dans 28 volumes de tampon d'incubation contenant du Tris (50mM), de l'acide ascorbique (0,1%), du chlorure de calcium (4mM) et de la pargylline (10µM), et dont le pH est ajusté à 7,4 avec de l'acide chlorhydrique.
La liaison avec la [$^3$H]mésulergine (1nM) est déterminée par incubation de 100 µl de suspension de membranes dans un volume final de 500 µl de milieu d'incubation.
Après une incubation de 30 min à 37°C suivie d'une incubation de 5 min entre 0 et 4°C, les membranes sont récupérées par filtration sur filtres Whatman GF/B™, préalablement traités pendant 30 min par de la polyéthylènimine à 0,05%, et lavées avec deux fois 1 ml de tampon Tris 50mM glacé dont le pH est ajusté à 7,4 avec de l'acide chlorhydrique.
Les filtres sont extraits dans le liquide de scintillation et la radioactivité est mesurée par scintigraphie liquide. La liaison spécifique de la [$^3$H]mésulergine est définie comme la quantité de radioactivité retenue sur les filtres et pouvant être inhibée par co-incubation avec la 5-hydroxytryptamine à 10µM. A une concentration de 1nM de [$^3$H]mésulergine la liaison spécifique représente 90% de la radioactivité totale récupérée sur le filtre.
Pour chaque concentration de composé étudié on détermine le pourcentage d'inhibition de la liaison avec la [$^3$H]mésulergine, puis la concentration $CI_{50}$, concentration qui inhibe 50% de la liaison.
Les composés de l'invention ont, dans cet essai, des $CI_{50}$ de 5 à 500 nM.

*In vivo*, l'activité centrale (du type $5HT_{1A}$) des composés de l'invention a été évaluée par leurs effets sur les "pointes PGO" (ponto-géniculo-occipitales) induites par la réserpine (test PGO-R) chez le chat, selon la méthode décrite par H. Depoortere, Sleep 1976, 3rd Europ. Congr. Sleep Res., Montpellier 1976, 358-361 (Karger, Basel 1977).
On administre des doses cumulatives de composés à étudier (de 0,1 à 3 mg/kg par voie intraveineuse) à des intervalles de temps de 30 min, 4 h après injection intrapéritonéale d'une dose de 0,75 mg/kg de réserpine, à des chats curarisés, sous ventilation artificielle. On recueille les activités électroencéphalographique et phasique (pointes PGO-R) à l'aide d'électrodes corticales et profondes (genouillé latéral).
Pour chaque dose de composé étudié on détermine le pourcentage de diminution du nombre de pointes PGO, puis la $DA_{50}$, dose active qui diminue de 50% ce nombre de pointes.

Pour les composés de l'invention les $DA_{50}$ sont inférieures à 0,3 mg/kg par la voie intraveineuse.

Enfin, l'activité antisérotoninergique (du type $5HT_2$) des composés de l'invention a été étudiée quant à leur effet sur l'inhibition des "head-twitches" (ébrouements de la tête) provoqués par le L-5-hydroxy-tryptophane (L-5-HTP) chez la souris, selon la méthode décrite par Corne et coll., *Br. J. Pharmacol.* (1962) **20** 106-120.
Les souris (mâles CD1, Charles River France (18 à 22 g de poids corporel) reçoivent les produits à étudier, à doses croissantes, ou le solvant, par voie intrapéritonéale ou orale, simultanément (voie i.p.) ou soixante minutes avant (voie orale) une injection de L-5-HTP à la dose de 250 mg/kg par voie sous-cutanée. Quarante-cinq minutes après cette injection de 5-HTP, le nombre d'ébrouements est compté, pour chaque souris, pendant une minute.
Pour chaque traitement, la moyenne des ébrouements, ainsi que le pourcentage de variation par rapport au lot témoin, sont calculés.
A partir de la courbe effet-dose, on détermine la $DA_{50}$ (dose active 50% ou dose qui diminue de 50% le nombre moyen d'ébrouements par rapport aux animaux témoins) par la méthode graphique de Miller et Tainter (*Proc. Soc. Exp. Biol. Med.* (1944) **57** 261).
Les $DA_{50}$ des composés de l'invention sont inférieures à 3 mg/kg par voie intrapéritonéale et sont de l'ordre de 1,5 mg/kg par voie orale.

Les résultats des essais montrent que les composés de l'invention ont une forte affinité pour les récepteurs sérotoninergiques de types $5HT_{1A}$, $5HT_{1D}$ et $5HT_{1C}$, ainsi qu'une certaine affinité pour les récepteurs $5HT_2$. *In vivo* ils possèdent des propriétés agonistes $5HT_{1A}$ et antagonistes $5HT_2$.

Ces résultats suggèrent que les composés sont utilisables pour le traitement de toutes affections liées à des dysfonctionnements des récepteurs sérotoninergiques de type $5HT_{1A}$, $5HT_{1D}$, $5HT_{1C}$ et/ou $5HT_2$, en particulier pour le traitement des états d'anxiété, de la dépression, des troubles du sommeil, des phobies, des troubles obsessionnels compulsifs, des troubles liés à l'alcoolisme, des troubles du comportement sexuel, pour la régulation de la prise de nourriture, et également pour le traitement des désordres vasculaires ou cardiovasculaires tels que la migraine et l'hypertension.

A cet effet ils peuvent être présentés sous toutes formes pharmaceutiques adaptées à l'administration entérale ou parentérale, en association avec des excipients appropriés, par exemple sous forme de comprimés, dragées, gélules, capsules, suppositoires, solutions ou suspensions buvables ou injectables, dosés pour permettre une administration journalière de 1 à 1000 mg de substance active.

## Revendications

1. Composé répondant à la formule générale (I)

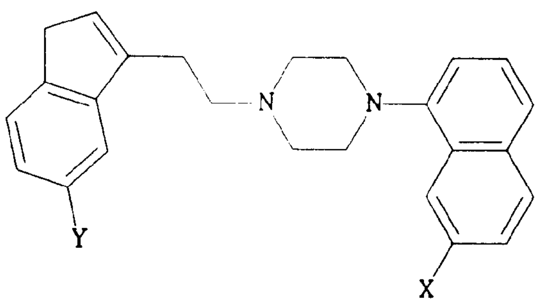

(I)

dans laquelle
X représente un atome d'hydrogène, un groupe alcoxy en $C_1$-$C_3$ ou un groupe cyclopropylméthoxy, et
Y représente un atome d'hydrogène ou un groupe méthoxy, à l'état de bases ou de sel d'addition.

2. Composé selon la revendication 1, caractérisé en ce que X représente un groupe alcoxy en $C_1$-$C_3$ et Y représente un groupe méthoxy.

3. Composé selon la revendication 2, caractérisé en ce que X représente un groupe méthoxy.

4. Procédé de préparation d'un composé selon la revendication 1, caractérisé en ce que,
ou bien
a) on traite un dérivé d'acide 1*H*-indène-3-acétique de formule générale (II)

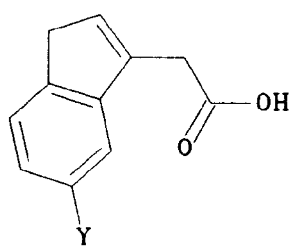

(II)

dans laquelle Y est tel que défini dans la revendication 1, avec un agent réducteur, pour former l'alcool de formule générale (III)

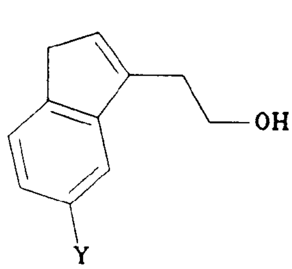

(III)

puis on traite cet alcool par le chlorure d'acide 4-méthylbenzènesulfonique, pour obtenir le dérivé de

formule générale (IV)

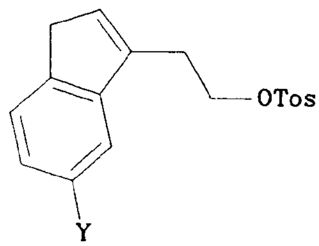

(IV)

et finalement on fait réagir ce dernier avec un dérivé de pipérazine de formule générale (V)

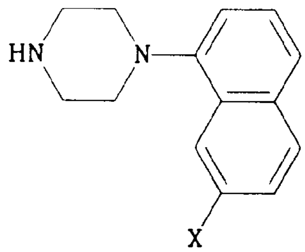

(V)

dans laquelle X est tel que défini dans la revendication 1,
ou bien
b) on fait réagir ledit dérivé d'acide 1*H*-indène-3-acétique de formule générale (II) d'abord avec le *N*,*N*'-carbonyldiimidazole, pour obtenir *in situ* l'imidazolide correspondant, puis on traite ce dernier avec ledit dérivé de pipérazine de formule générale (V) pour obtenir l'amide de formule générale (VI)

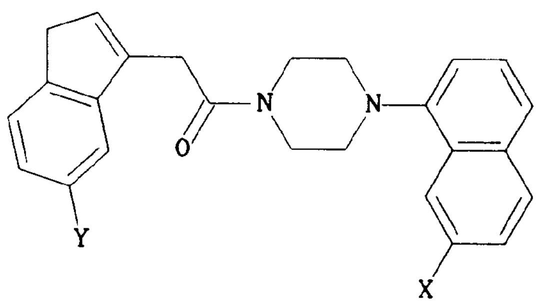

(VI)

et finalement on réduit ce dernier au moyen d'un agent réducteur.

**5.** Médicament caractérisé en ce qu'il consiste en un composé selon la revendication 1.

**6.** Composition pharmaceutique caractérisée en ce qu'elle contient un composé selon la revendication 1, associé à un excipient.

Office européen des brevets

# RAPPORT DE RECHERCHE EUROPEENNE

Numero de la demande
EP 95 40 0304

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | FR-A-2 318 635 (E.R. SQUIBB & SONS)<br>*Document entier: particulièrement examples 2,4,5,29-32,42-46,78-80*<br>--- | 1-6 | C07D295/08<br>A61K31/495 |
| A | WO-A-94 00441 (PIERREL S.P.A.)<br>*Page 51-68. révendications*<br>--- | 1-6 | |
| D,A | EP-A-0 490 772 (ADIR ET COMPAGNIE)<br>*Document entier*<br>----- | 1-6 | |
| | | | DOMAINES TECHNIQUES RECHERCHES (Int.Cl.6) |
| | | | C07D |

Le présent rapport a été établi pour toutes les revendications

| Lieu de la recherche | Date d'achèvement de la recherche | Examinateur |
|---|---|---|
| LA HAYE | 6 Mars 1995 | Luyten, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)